# EUROPEAN PATENT SPECIFICATION

(11) **EP 4 534 048 B1**
(45) Date of publication and mention of the grant of the patent: **28.01.2026**
(21) Application number: 23201766.5
(22) Date of filing: 05.10.2023
(51) Int. Cl.: A61F 2/38, A61B 17/88, A61F 2/46

(54) **PATELLA IMPACTION DEVICE AND PATELLA PREPARATION SET**
PATELLAIMPAKTIONSVORRICHTUNG UND PATELLAVORBEREITUNGSSET
DISPOSITIF D'IMPACTION ROTULE ET ENSEMBLE DE PRÉPARATION DE ROTULE

(43) Date of publication of application: 09.04.2025
(73) Proprietor: Aesculap AG, 78532 Tuttlingen (DE)
(72) Inventor: Snauwaert, Eliott, 52000 Chaumont (FR)
(74) Representative: DREISS Patentanwälte PartG mbB

(56) References cited:
- FR-A1- 2 732 210
- US-A- 6 010 509
- US-A1- 2017 086 989
- US-A1- 2018 014 839

## Description

The invention relates to a patella impaction device known from US 6 010 509 A and according to the preamble of claim 1.

During a total knee replacement or total knee arthroplasty ("TKA") surgery, the posterior portion of a patella may be resected for implanting with a patella implant. The step of resecting the patella requires the use of a patella clamping device for retaining or holding the patella during cutting. US 2006/0161165 A1 proposes to provide a pincer-like structure with two mutually movable handle parts, one handle part being connected to a first clamping section engaging the skin / subcutaneous tissues overlying the anterior of a patella and the other handle part being connected to a second clamping section engaging the posterior of a patella, thus providing a clamping action along an antero-posterior axis of the patella.

Patella impaction devices are also known in the art and used for pressing a patella implant against a resected patella during a hardening process of a cement which connects the patella implant to the resected patella.

The choice of the size and the geometry of a specific patella implant depends on the size and geometry of the knee of the patient.

One object of the present invention is to provide a patella impaction device and a patella preparation set which allow for a reliable impaction of patella implants having a different geometry.

This object is solved by the patella impaction device with the features of claim 1. This object is also solved by a patella preparation set according to claim 9.

Preferred embodiments are defined in the dependent claims.

The patella impaction device comprises a shell-shaped clamping element which is mounted on a holding structure of the first clamp in a floating manner such that the clamping element is movable at least along one axis of movement within a plane extending perpendicular to the pressure axis. This movement allows for an alignment of a peak point axis of a specific patella implant with a corresponding center or lowest point of the shell-shaped clamping element without having to adjust the position of the holding structure.

In one embodiment, the clamping element is movable at least along a medio-lateral axis allowing for alignment of a peak point axis of a patella implant along the medio-lateral axis.

In a further embodiment, the clamping element is movable at least along a proximal-distal axis allowing for alignment of the peak point axis of a patella implant along the proximal-distal axis.

In a preferred embodiment, the clamping element is movable both along the medio-lateral axis and along the proximal-distal axis allowing for an alignment of the peak point axis of a patella implant within a plane extending perpendicular to the pressure axis.

The holding structure comprises at least one guiding element for guiding the clamping element. The guiding element defines at least one axis of movement of the clamping element. For example, the holding structure comprises two distinct guiding elements being arranged parallel to each other.

According to the invention, the clamping element comprises a guiding slot for sliding engagement of the at least one guiding element. The guiding slot and the at least one guiding element are in a form-fit engagement in a direction parallel to the pressure axis and thus attaching the clamping element to the holding structure in a movable manner.

According to one aspect of the invention, the guiding slot extends along a length which is shorter than a guiding length of the at least one guiding element such that the clamping element is movable along an extension axis of the guiding element.

According to an alternative or further aspect of the invention, the guiding slot has a width which is greater than a width of the at least one guiding element such that the clamping element is movable in a direction perpendicular to an extension axis of the guiding element.

In one embodiment, the holding structure has a U-shape which is stable and allows for a compact integration of at least one guiding element.

Preferably, the clamping element is made of plastic material to preserve the geometry of a surface of a patella implant during the impaction process. Preferably, the holding structure and/or at least one guiding element are made from metal material.

In a preferred embodiment, the device comprises a second clamp configured to be located on the anterior of the resected patella, and further comprising a pressing device for pressing the first clamp and the second clamp towards each other. The pressing device may be a manual clamp, e.g. of a pincer-type.

In one embodiment, the holding structure of the first clamp and the pressing device are permanently connected to each other allowing for a simple and robust device.

In another embodiment, the holding structure and the pressing device are removably connectable to each other by means of a connector and allowing for the holding structure to be used with different pressing devices or for a pressing device to be used with different holding structures.

The invention also relates to a patella preparation set comprising a patella impaction device as described above and further comprising at least two patella implants, the two patella implants being alternatively being connectable to a resected patella and having identical connection elements for being connecting to the resected patella, wherein the peak point axes of the two patella implants are offset to each other with respect to their medio-lateral axes. Independent of the choice of either one of these two patella implants, the impaction of a selected patella implant and a resected patella can take place along a pressure axis which is aligned with the peak point axis of the selected patella implant.

Further features and advantages are the subject of the following description and of the diagrammatic illustration of embodiments.

In the drawing:
- Figure 1: shows a perspective view of one embodiment of a patella impaction device engaging a patella implant;
- Figure 2: shows a sectional view according to sectional plane II - II indicated in Fig. 1;
- Figure 3: shows a side view according to arrow III indicated in Fig. 1;
- Figure 4: shows a rear view of the patella implant of Fig. 1;
- Figure 5: shows a sectional view according to sectional plane V - V indicated in Fig. 4;
- Figure 6: shows shows a rear view of another patella implant; and
- Figure 7: shows a sectional view according to sectional plane VII - VII indicated in Fig. 6.

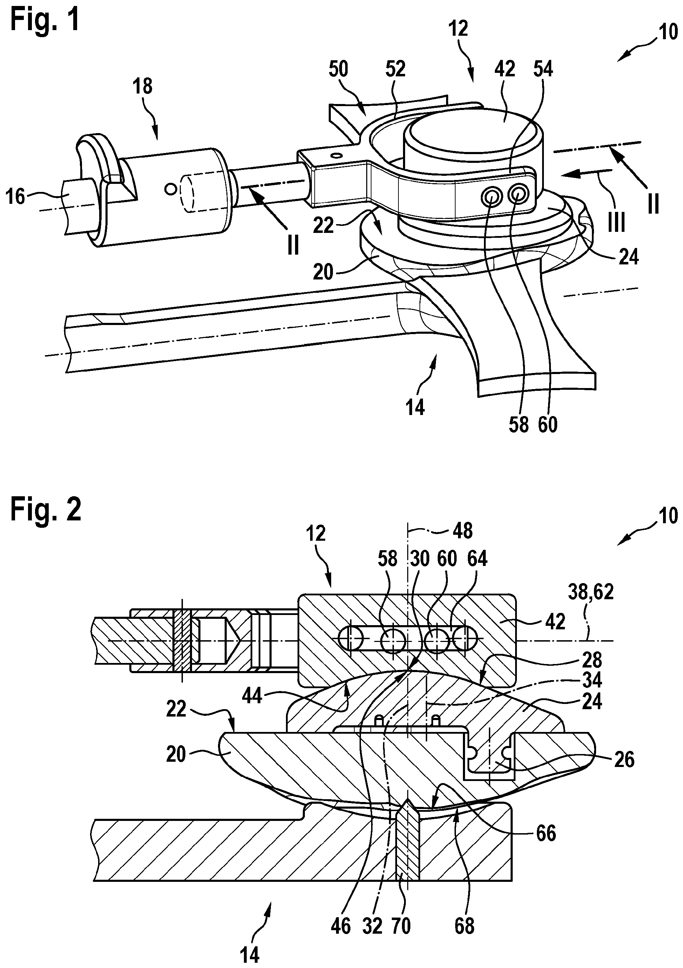

According to Fig. 1, a patella impaction device 10 comprises a first clamp 12 and a second clamp 14 which can be pressed towards each other by means of a pressing device 16. The pressing device is illustrated in a schematic and partial manner only; an example of a pressing device 16 is a clamp with two handles or grips to be manipulated by a hand of a surgeon.

The first clamp 12 is releasably connectable or connected to the pressing device 16 by means of a connector 18 which by way of example may incorporate a threading or snap-fit connection.

The impaction device 10 is used in connection with a resected patella 20 having a resection plane 22 and with a patella implant 24 having connection elements 26 to be connected with the resected patella 20.

By way of example, Fig. 1 shows a right knee patella; a lateral side corresponds with a left side of the drawing and a medial side corresponds with a right side of the drawing. A tibia would be arranged at the front of the drawing and a femur would be arranged in the background of the drawing.

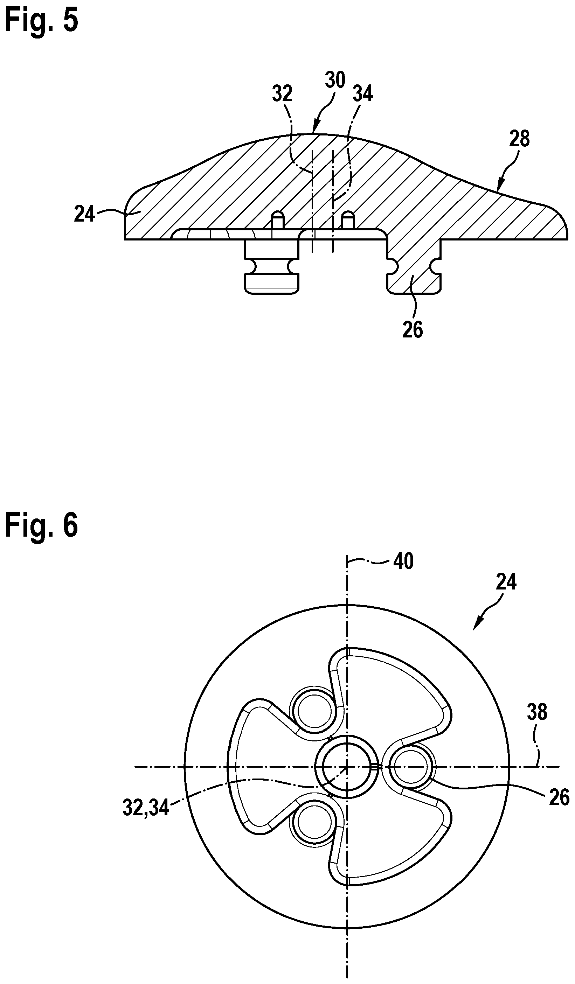

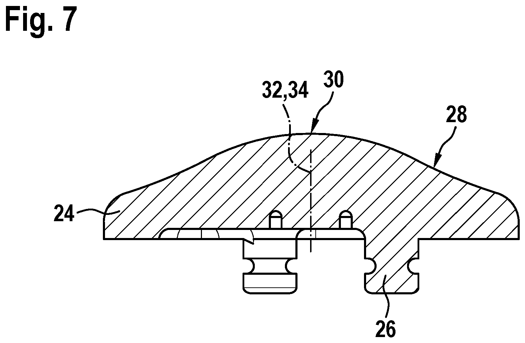

The patella implant 24 comprises a dome-shaped surface 28 which comprises a peak point 30 of a peak point axis 32. In the example shown in Fig. 1 to 5, the peak point axis 32 is offset to a central axis 34 of the patella implant 24. In the example of a patella implant 24 shown in Fig. 6 and 7, the peak point axis 32 and the central axis 34 are aligned with each other.

The central axis 34 is defined by an intersection point of an extension of the patella implant along a medio-lateral axis 38 and along a proximal-distal axis 40.

The anterior face 28 of the patella implant is dome-shaped and is complementary to a shell-shaped clamping element 42 having a shell 44 with a center or lowest-point 46 which defines a pressure axis 48.

The patella impaction device 10 comprises a U-shaped holding structure 50 with two holding arms 52, 54 extending parallel to each other and delimiting a holding space 56 in which the clamping element 42 is positioned in a movable manner.

The holding arms 52, 54 support two bolt-shaped guiding elements 58, 60 which are arranged within the holding space 56 and which are connected to the holding arms 52, 54. The guiding elements 58, 60 define a plane 62 which is perpendicular to the pressure axis 48. The clamping element 42 is movable within the plane 62 and with respect to the holding structure 50 by means of a guiding slot 64 which surrounds the guiding elements 58, 60.

The guiding slot 64 has a width which is greater than a combined width of the two guiding elements 58, 60, such that the clamping element 42 is movable along a medio-lateral axis 38, see Fig. 2.

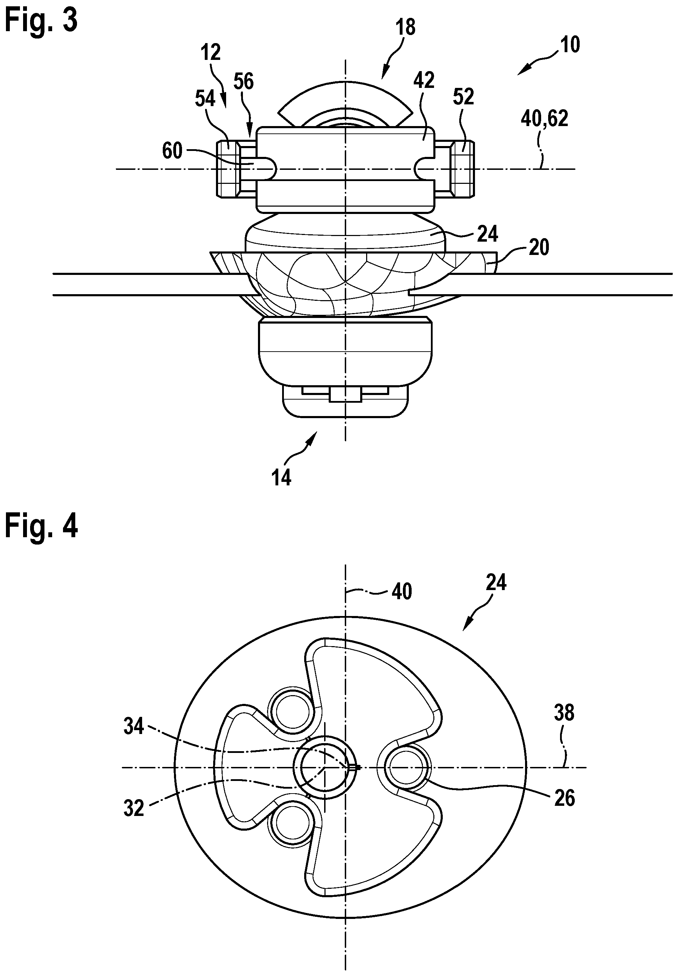

The guiding slot 64 extends along a length which is shorter than a guiding length of the at least one guiding element 58, 60, such that the clamping element 42 is movable along a proximal-distal axis 40, see Fig. 3.

The guiding slot 64 has a width which is greater than a combined width of the two guiding elements 58, 60, such that the clamping element 42 is movable along a medio-lateral axis 38, see Fig. 2.

The second clamp 14 comprises a shell-shaped surface 66 to be pressed against an anterior surface 68 of the resected patella 20. The second clamp 14 may comprise a spike 70 engaging with the surface 68.

When impacting the patella implant 24 to the resected patella 20, the clamping element 42 and its shell 44 are movable within plane 62 in order to self-align the center or lowest-point 46 of the shell 44 and the corresponding pressure axis 48 on the one hand with the peak point 30 of the peak point axis 32 of the patella implant 24 on the other hand.

By way of example, if the patella implant 24 shown in Fig. 6 and 7 was used in a situation as shown in Fig. 1, 2 and 3, the peak point axis 32 of the patella implant 24 would be shifted and be aligned with the central axis 34. Assuming that the holding structure 50 remains in the same position as shown in Fig. 1, 2 and 3, the clamping element 42 would be shifted along the medio-lateral axis 38 accordingly (to the right in Fig. 2).

## Claims

1. Patella impaction device (10), comprising a first clamp (12) configured to be pressed along a pressure axis (48) and onto a dome-shaped surface (28) of a patella implant (24) to be connected to a resected patella (20), wherein the first clamp (12) comprises a shell-shaped clamping element (42) which is mounted on a holding structure (50) of the first clamp (12) in a floating manner and allowing the clamping element (42) to move at least along one axis of movement (38, 40) within a plane (62) extending perpendicular to the pressure axis (48), wherein the holding structure (50) comprises at least one guiding element (58, 60) for guiding the clamping element (42), wherein the at least one guiding element extends along an extension axis (40), **characterized in**
**that** the clamping element (42) comprises a guiding slot (64) for sliding engagement of the at least one guiding element (58, 60), wherein the guiding slot (64) and the at least one guiding element (58, 60) are in a form-fit engagement in a direction parallel to the pressure axis (48) and thus attaching the clamping element (42) to the holding structure (50) in a movable manner, wherein the guiding slot (64) extends along a length which is shorter than a guiding length of the at least one guiding element (58, 60) such that the clamping element (42) is movable along the extension axis of the at least one guiding element (58, 60) and/or wherein the guiding slot (64) has a width which is greater than a width of the at least one guiding element (58, 60) such that the clamping element (42) is movable in a direction perpendicular to the extension axis of the at least one guiding element (58, 60).

2. Patella impaction device (10) according to claim 1, wherein the clamping element (42) is movable at least along a medio-lateral axis (38).

3. Patella impaction device (10) according to one of the preceding claims, wherein the clamping element (42) is movable at least along a proximal-distal axis (40).

4. Patella impaction device (10) according to one of the preceding claims, wherein the holding structure (50) comprises two distinct guiding elements (58, 60) being arranged parallel to each other.

5. Patella impaction device (10) according to one of the preceding claims, wherein the holding structure (50) has a U-shape.

6. Patella impaction device (10) according to one of the preceding claims, wherein the clamping element (42) is made of plastic material.

7. Patella impaction device (10) according to one of the preceding claims, wherein the device (10) further comprises a second clamp (14) configured to be located on the anterior of the resected patella (20) and further comprising a pressing device (16) for pressing the first clamp (12) and the second clamp (14) towards each other.

8. Patella impaction device (10) according to claim 7, wherein the holding structure (50) of the first clamp (12) and the pressing device (16) are permanently connected to each other and/or in that the holding structure (50) and the pressing device (16) are removably connectable to each other by means of a connector (18).

9. Patella preparation set comprising a patella impaction device (10) according to one of the preceding claims and at least two patella implants (24), the two patella implants (24) being alternatively being connectable to a resected patella (20) and having identical connection elements (26) for being connecting to the resected patella (20), wherein peak point axes (32) of the two patella implants (24) are offset to each other with respect to their medio-lateral axes (38).

## Patentansprüche

1. Patella-Impaktionsvorrichtung (10), umfassend eine erste Klemme(12), die konfiguriert ist, um entlang einer Druckachse (48) und auf eine kuppelförmige Oberfläche (28) eines Patella-Implantats (24) gedrückt zu werden, das mit einer resezierten Patella (20) zu verbinden ist, wobei die erste Klemme (12) ein schalenförmiges Klemmelement (42) umfasst, das an einer Haltestruktur (50) der ersten Klemme (12) schwimmend montiert ist und dem Klemmelement (42) ermöglicht, sich mindestens entlang einer Bewegungsachse (38, 40) innerhalb einer Ebene (62) zu bewegen, die sich senkrecht zu der Druckachse (48) erstreckt, wobei die Haltestruktur (50) mindestens ein Führungselement (58, 60) zum Führen des Klemmelements (42) umfasst, wobei sich das mindestens eine Führungselement entlang einer Erstreckungsachse (40) erstreckt,
**dadurch gekennzeichnet, dass** das Klemmelement (42) einen Führungsschlitz (64) für gleitenden Eingriff des mindestens einen Führungselements (58, 60) umfasst, wobei der Führungsschlitz (64) und das mindestens eine Führungselement (58, 60) in einem formschlüssigen Eingriff in einer Richtung parallel zu der Druckachse (48) stehen und somit das Klemmelement (42) auf bewegbare Weise an der Haltestruktur (50) befestigen, wobei sich der Führungsschlitz (64) entlang einer Länge erstreckt, die derart kürzer als eine Führungslänge des mindestens einen Führungselements (58, 60) ist, dass das Klemmelement (42) entlang der Erstreckungsachse des mindestens einen Führungselements (58, 60) bewegbar ist und/oder wobei der Führungsschlitz (64) eine Breite aufweist, die derart größer als eine Breite des mindestens einen Führungselements (58, 60) ist, dass das Klemmelement (42) in einer Richtung senkrecht zu der Erstreckungsachse des mindestens einen Führungselements (58, 60) bewegbar ist.

2. Patella-Impaktionsvorrichtung (10) nach Anspruch 1, wobei das Klemmelement (42) mindestens entlang einer medio-lateralen Achse (38) bewegbar ist.

3. Patella-Impaktionsvorrichtung (10) nach einem der vorstehenden Ansprüche, wobei das Klemmelement (42) mindestens entlang einer proximaldistalen Achse (40) bewegbar ist.

4. Patella-Impaktionsvorrichtung (10) nach einem der vorstehenden Ansprüche, wobei die Haltestruktur (50) zwei unterschiedliche Führungselemente (58, 60) umfasst, die parallel zueinander angeordnet sind.

5. Patella-Impaktionsvorrichtung (10) nach einem der vorstehenden Ansprüche, wobei die Haltestruktur (50) eine U-Form aufweist.

6. Patella-Impaktionsvorrichtung (10) nach einem der vorstehenden Ansprüche, wobei das Klemmelement (42) aus Kunststoffmaterial hergestellt ist.

7. Patella-Impaktionsvorrichtung (10) nach einem der vorstehenden Ansprüche, wobei die Vorrichtung (10) ferner eine zweite Klemme (14) umfasst, die konfiguriert ist, um an der Vorderseite der resezierten Patella (20) angeordnet zu werden, und ferner umfassend eine Pressvorrichtung (16) zum Pressen der ersten Klemme (12) und der zweiten Klemme (14) aufeinander zu.

8. Patella-Impaktionsvorrichtung (10) nach Anspruch 7, wobei die Haltestruktur (50) der ersten Klemme (12) und die Pressvorrichtung (16) dauerhaft miteinander verbunden sind und/oder wobei die Haltestruktur (50) und die Pressvorrichtung (16) mittels eines Verbinders (18) lösbar miteinander verbindbar sind.

9. Patella-Vorbereitungssatz, umfassend eine Patella-Impaktionsvorrichtung (10) nach einem der vorstehenden Ansprüche und mindestens zwei Patella-Implantate (24), wobei die zwei Patella-Implantate (24) alternativ mit einer resezierten Patella (20) verbindbar sind und identische Verbindungselemente (26) zum Verbundenwerden mit der resezierten Patella (20) aufweisen, wobei Scheitelpunktachsen (32) der zwei Patella-Implantate (24) zueinander bezüglich ihrer medio-lateralen Achsen (38) versetzt sind.

## Revendications

1. Dispositif d'impaction de rotule (10), comprenant une première pince (12) conçue pour être pressée le long d'un axe de pression (48) et sur une surface en forme de dôme (28) d'un implant de rotule (24) à relier à une rotule réséquée (20), dans lequel la première pince (12) comprend un élément de serrage en forme de coquille (42) qui est monté sur une structure de maintien (50) de la première pince (12) de manière flottante et permettant à l'élément de serrage (42) de se déplacer au moins le long d'un axe de mouvement (38, 40) dans un plan (62) s'étendant perpendiculairement à l'axe de pression (48), dans lequel la structure de maintien (50) comprend au moins un élément de guidage (58, 60) pour guider l'élément de serrage (42), dans lequel l'au moins un élément de guidage s'étend le long d'un axe d'extension (40),
**caractérisé en ce que** l'élément de serrage (42) comprend une fente de guidage (64) pour la mise en prise coulissante de l'au moins un élément de guidage (58, 60), dans lequel la fente de guidage (64) et l'au moins un élément de guidage (58, 60) sont dans une mise en prise de forme dans une direction parallèle à l'axe de pression (48) et fixant ainsi l'élément de serrage (42) à la structure de maintien (50) d'une manière mobile, dans lequel la fente de guidage (64) s'étend le long d'une longueur inférieure à une longueur de guidage de l'au moins un élément de guidage (58, 60), de telle sorte que l'élément de serrage (42) est mobile le long de l'axe d'extension de l'au moins un élément de guidage (58, 60) et/ou dans lequel la fente de guidage (64) présente une largeur supérieure à une largeur de l'au moins un élément de guidage (58, 60), de telle sorte que l'élément de serrage (42) est mobile dans une direction perpendiculaire _{à} l'axe d'extension de l'au moins un élément de guidage (58, 60).

2. Dispositif d'impaction de rotule (10) selon la revendication 1, dans lequel l'élément de serrage (42) est mobile au moins le long d'un axe médio-latéral (38).

3. Dispositif d'impaction de rotule (10) selon l'une des revendications précédentes, dans lequel l'élément de serrage (42) est mobile au moins le long d'un axe proximal-distal (40).

4. Dispositif d'impaction de rotule (10) selon l'une des revendications précédentes, dans lequel la structure de maintien (50) comprend deux éléments de guidage distincts (58, 60) agencés parallèlement l'un à l'autre.

5. Dispositif d'impaction de rotule (10) selon l'une des revendications précédentes, dans lequel la structure de maintien (50) présente une forme en U.

6. Dispositif d'impaction de rotule (10) selon l'une des revendications précédentes, dans lequel l'élément de serrage (42) est en matière plastique.

7. Dispositif d'impaction de rotule (10) selon l'une des revendications précédentes, dans lequel le dispositif (10) comprend en outre une seconde pince (14) conçue pour être située sur la partie antérieure de la rotule réséquée (20) et comprenant en outre un dispositif de pression (16) pour presser la première pince (12) et la seconde pince (14) l'une vers l'autre.

8. Dispositif d'impaction de rotule (10) selon la revendication 7, dans lequel la structure de maintien (50) de la première pince (12) et le dispositif de pression (16) sont reliés de manière permanente l'un à l'autre et/ou dans lequel la structure de maintien (50) et le dispositif de pression (16) peuvent être reliés de manière amovible l'un à l'autre au moyen d'un connecteur (18).

9. Ensemble de préparation de rotule comprenant un dispositif d'impaction de rotule (10) selon l'une des revendications précédentes et au moins deux implants de rotule (24), les deux implants de rotule (24) pouvant alternativement être reliés à une rotule réséquée (20) et présentant des éléments de liaison identiques (26) pour être reliés à la rotule réséquée (20), dans lequel des axes de point de crête (32) des deux implants de rotule (24) sont décalés l'un par rapport à l'autre par rapport à leurs axes médio-latéraux (38).
